# EUROPEAN PATENT SPECIFICATION

(11) **EP 2 012 692 B1**
(45) Date of publication and mention of the grant of the patent: **08.12.2010**
(21) Application number: 07760689.5
(22) Date of filing: 16.04.2007
(51) Int. Cl.: A61B 17/70

(54) **Revision fixation plate**
Fixationsplatte für Revision
Plaque de fixation secondaire

(30) Priority: 26.04.2006 US 411751
(43) Date of publication of application: 14.01.2009
(73) Proprietor: Warsaw Orthopedic, Inc., Warsaw, Indiana 46581 (US)
(72) Inventor: BAKER, Douglas N., Collierville, Tennessee 38017 (US); CAPOTE, Marco D., Lafayette, CO 80026 (US); DICKINSON, Charles A., Millington, Tennessee 38053 (US); TAYLOR, Harold S., Memphis, Tennessee 38112 (US); BISCUP, Robert S., Fort Lauderdale, Florida 33316 (US); LEROUX, Clayton G., Suite 208, Westlake OH 44145 (US)
(74) Representative: Hutchinson, Glenn Stanley
(86) International application number: PCT/US2007/066679
(87) International publication number: WO 2007/127632

(56) References cited:
- EP-A- 1 205 154
- US-A1- 2003 163 132
- US-A1- 2003 225 409
- US-B1- 6 273 914
- US-B1- 6 379 354

## Description

### BACKGROUND

The present application relates generally to a fixation system for the treatment of the skeletal system. More particularly, the present invention may be applied to treatment of the human spine.

Spinal fusion is performed to prevent motion between mobile segments of the spine. A variety of reasons exist for performing spinal fusion. The spine may be unstable due to a traumatic injury, surgery, or invasion and destruction of the vertebrae by tumor. Continued motion of particular segments of the spine may cause overgrowth of joint and ligamentous tissue which, in turn, may compress the spinal cord or its nerves. The curvature of the spine may become abnormal and cause deformity or neurological problems. In these instances, it may be desirable to prevent spinal motion at the affected levels.

The spine is composed of individual bones, or vertebrae, stacked on top of each other in a column. Each vertebra includes a cylindrical vertebral body, which participates in weight bearing, and an arch of bone (comprising the lamina and spinous process) which protects the spinal cord and its covering. The bony arch is connected to the vertebral body by two small columns of bone, referred to as the pedicles. The circular canal between the body, the arch, and the pedicles houses the spinal cord and is called the spinal canal. Between adjacent vertebral bodies lie the intervertebral discs. These are cartilaginous structures that function as shock absorbers for the spine. Facet joints connect the bony arches of the spine and permit spinal motion between adjacent vertebrae.

Spinal instrumentation is employed as an adjunct to successful spinal fusion. The instrumentation immobilizes the spine while the body forms new, solid bone. Spinal fusion usually is performed by surgically exposing the area of the spine to be fused and thereafter preparing the exposed bone by removing soft tissue and ligaments so new bone can form over the area. After the surgical site has been prepared, an autogenic bone graft have been developed in an attempt to avoid the problems associated with acquiring a bone graft implant. Regardless of the type of implant that is used, the chances of achieving a successful fusion are enhanced if motion in the area is minimized or prevented while new bone forms. Further, even when an initial fusion surgery is successful, adjacent spine levels may be become affected and need instrumentation to promote a fusion at the adjacent level. In such situation is desirable to revise the initial surgical procedure.

US 2003/0225409 discloses a spinal plate extender system including circular apertures for fixation to the underlying vertebra. US6273914 discloses a spinal implant according to the preamble of claim 1, in which a link has a slot at the first end and an aperture at a second end configured to facilitate secure maintenance of an adapter to the link.

Although there have been advances in this area, there remains a need for improved stabilization systems for use in skeletal fixation and boney fusion procedures.

### SUMMARY OF THE INVENTION

The present application relates generally to fixation of the skeletal system and is defined by independent claim 1. Preferred embodiments are defined by the dependent claims.

In one embodiment, a system is provided for extending a first implanted spinal fixation element to one or more adjacent vertebrae. In one aspect the system includes an elongated extension member having at least one locking projection for engaging the first implanted spinal system to inhibit rotation between the two components.

In yet a further aspect, the present invention provides a spinal fixation system for joining a first vertebra to a second vertebra. The fixation system comprising an elongated fixation member, a first bone anchor, a second bone anchor and at least one coupler for joining one of the first bone anchor or second bone anchor to the fixation member, wherein the coupler receives the bone anchor in an internal passage and has an outer threaded surface.

There is also described an exemplary method for revising a first implanted spinal fixation system attached to the spine with at least one bone anchor and a dual action coupling element. The method includes providing an extension member and a locking member, positioning one end of the extension member adjacent the dual action coupling element and locking the locking member to the dual action coupling element such that dual action coupling element participates in locking the first implanted spinal fixation system to the spine and locking the extension member to the first implanted spinal fixation system.

Further aspects, forms, embodiments, objects, features, benefits, and advantages of the present invention shall become apparent from the detailed drawings and descriptions provided herein.

### BRIEF DESCRIPTION OF THE DRAWINGS

Fig. 1 is a perspective view of a revision system according to one aspect of the present invention implanted in the spine.
Fig. 2A is a partially exploded perspective view of an implanted system and a revision system according to one aspect of the present invention.
Fig. 2B is a perspective view of an assembled combination of the components of Fig. 1.
Fig. 2C is a partial cross-sectional view taken along line 2C-2C of Fig. 2B.
Fig. 3A is a partially exploded perspective view of an implanted system and a further embodiment of a revision system according to another aspect of the present invention.
Fig. 3B is a perspective view of an assembled combination of the components of Fig. 3A in an unlocked condition.
Fig. 3C is a perspective view of an assembled combination of the components of Fig. 3A in a locked condition.
Fig. 4A is a partially exploded perspective view of an implanted system and a further embodiment of a revision system according to another aspect of the present invention.
Fig. 4B is a perspective view of an assembled combination of the components of Fig. 4A.
Fig. 4C is a partial cross-sectional view taken along line 4C-4C of Fig. 4B.
Fig. 5A is a partially exploded perspective view of an implanted system and a further embodiment of a revision system according to another aspect of the present invention.
Fig. 5B is a perspective view of an assembled combination of the components of Fig. 5A.
Fig. 5C is a partial cross-sectional view taken along line 5C-5C of Fig. 5B.
Fig. 6A is a partially exploded perspective view of an implanted system and a further embodiment of a revision system according to another aspect of the present invention.
Fig. 6B is a perspective view of an assembled combination of the components of Fig. 6A.
Fig. 7A is a partially exploded perspective view of a fixation system according to another aspect of the present invention.
Fig. 7B is an assembled perspective view of the fixation system of Fig. 7A.
Fig. 8 is a partially exploded perspective view of a further embodiment of a fixation system.
Fig. 9 is a partially exploded perspective view of a further embodiment of a fixation system.

### DETAILED DESCRIPTION OF THE ILLUSTRATED EMBODIMENTS

For the purposes of promoting an understanding of the principles of the invention, reference will now be made to the embodiments, or examples, illustrated in the drawings and specific language will be used to describe the same. It will nevertheless be understood that no limitation of the scope of the invention is thereby intended. Any alternations and further modifications in the described embodiments, and any further applications of the principles of the invention as described herein are contemplated as would normally occur to one skilled in the art to which the invention relates.

Spinal fixation systems, such as rod/screw systems and plate/screw systems, are often used to at least partially stabilize the spine to reduce movement between adjacent vertebrae. In some patients there is a need to address continued degradation of the spine near the previously implanted spiral fixation system. In these circumstances, it is desirable to have a revision fixation system that may be added onto the previously implanted fixation system to extend the composite system to one or more near by spinal levels.

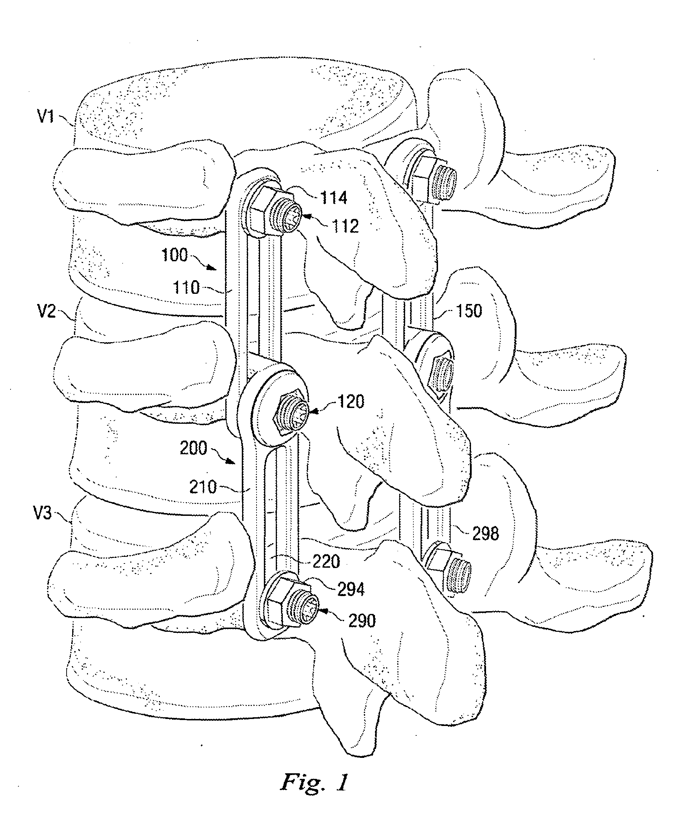

Referring now to Figs. 1-2C, there is shown an embodiment of a revision fixation plate system 200 in combination with a previously implanted spinal fixation system 100. The previously implanted fixation system 100 includes a plate 110 extending between vertebrae V1 and V2 having an internal slot 130. A bolt 120 extends through slot 130 into the pedicle of vertebra V2 and is initially coupled to the plate 110 by an internally threaded nut similar to nut 114. In a similar manner, a bolt 112 similar to bolt 120, extends through slot 130 into the pedicle of the vertebrae V1 and is coupled to the plate 110 by an internally threaded nut 114. Bone bolt 120 includes a bone engaging threaded shaft 122, a enlarged seat area 124 and an externally threaded coupling shaft 126 having machine threads to receive a fastener such as nut 114. In the illustrated embodiment, disposed between seat 124 and plate 110 is an enlarged washer 128 adapted to engage the underside of the plate. In the illustrated embodiment, the bone bolt 120 has an external drive pattern on the enlarged seat 124 and an internal hex drive pattern formed adjacent threads 126. The slot 130 in the plate 110 is defined by opposing elongated side walls 132, 134 and opposing end walls 136, 138. The top of the walls forming the slot 130 is chamfered or rounded to form surface 137 and the bottom of the walls are also chamfered to form surface 135. In the embodiment illustrated in Fig. 1, there is a second previously implanted spinal fixation system 150 extending between V1 and V2 on the opposite side of the spinous processes.

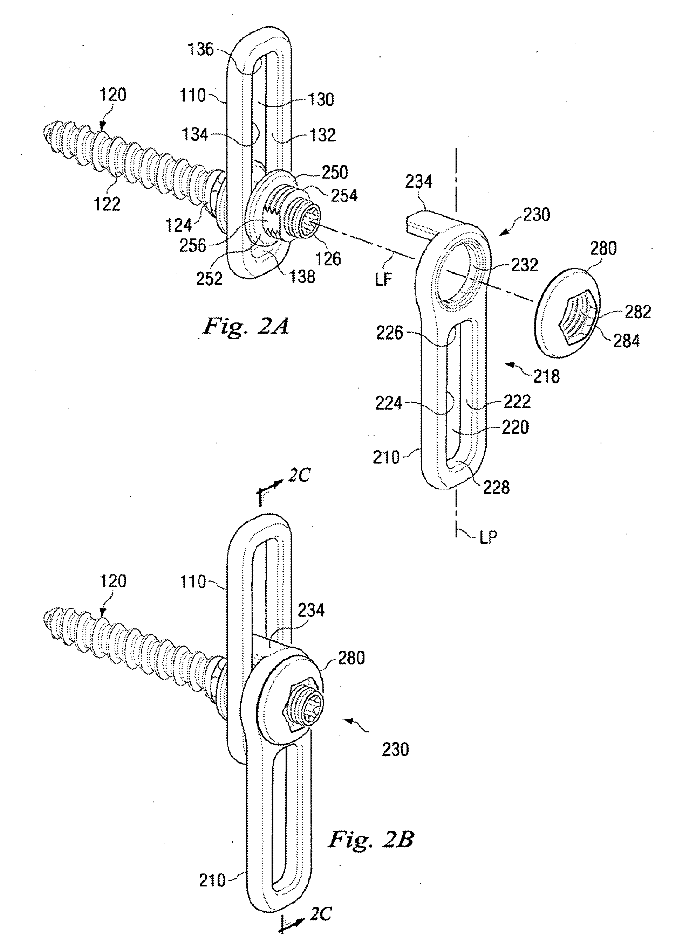

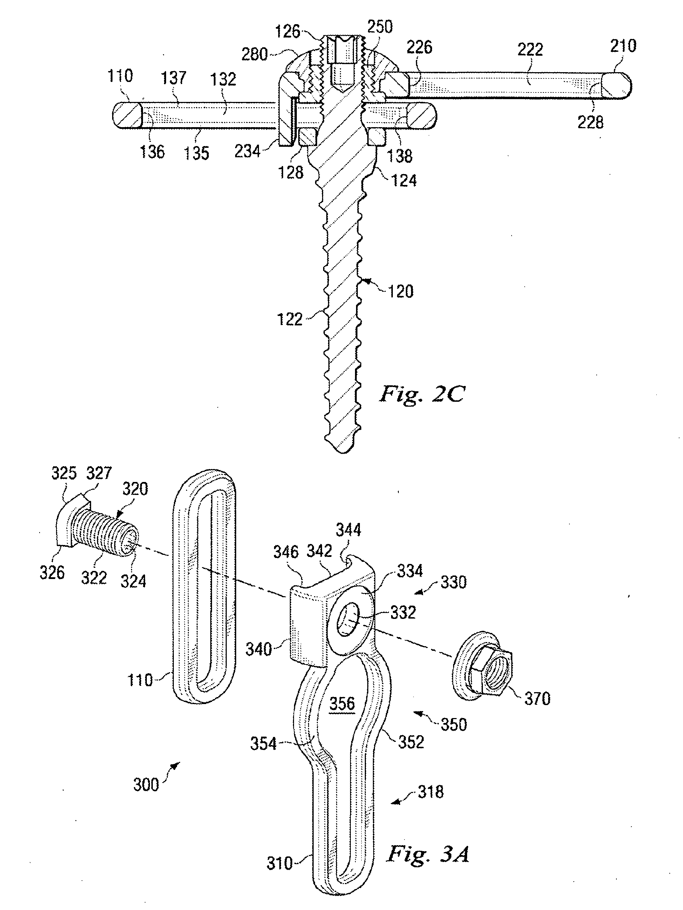

In the illustrated embodiment, an elongated extension system 200 is shown in combination with the previously implanted fixation system 100. The extension system includes a plate 210 having a fixation portion 218 with a slot 220 defined by opposed elongated side walls 222, 224 and end walls 226, 228. The Extension plate 210 also includes a connection portion 230 adapted for coupling to a previously implanted fixation system. Connection portion 230 includes a substantially cylindrical aperture 232 and a projecting flange 234 extending along axis LF transverse to the longitudinal axis LP of the plate. Projecting flange 234 has a width that substantially matches the width of the slot 130 and/or the length of the end walls 136, 138 to lock the extension plate 210 to the plate 110 to inhibit rotation therebetween. Further, as shown in Fig. 2C, the projection has a length transverse to the longitudinal axis of the plate 210 that is greater than the thickness from the top coupling surface to the bottom bone engaging surface of the plate 110.

The extension plate system 200 further includes a coupling member 250 and a cooperating internally threaded nut 280 to join the plate 210 to the pilate 110. Coupling member 250 has an enlarged flange 252 having a diameter larger than the width of slot 130 such that the flange engages plate 110. Coupling member 250 has an internally threaded bore configured to threadedly engage externally threaded shaft 126 of the bone bolt 120 and an external drive surface 256 and an opposing drive surface (not shown). The drive surface 256 allows a tool to engage the coupling member 250 and advance it along threaded shaft 126. The exterior of the coupling member 250 has a series of external threads interrupted by the drive surface 256. Threaded nut 280 includes an internally threaded aperture 282 and an internal drive pattern 284. The internally threaded aperture 282 is configured for threaded engagement with the external threads 254 of the coupling member.

In the assembled configuration shown in Figs. 2B and 2C, the extension plate 210 is positioned in substantial longitudinal alignment with the previously implanted fixation plate 110. The previously installed coupling member 250 and projecting portion of threaded shaft 126 are received within aperture 232 at the connection portion of the plate. Aperture 232 has a stepped lower surface as best seen in Fig. 2C sized to matingly receive the flange 252. With the coupling member positioned in aperture 232, projection 234 extends into and through slot 130 to lock the alignment of plate 210 with the alignment of plate 110. Projection 234 is sized to substantially mate with the walls of the slot 130 to prevent rotation between plate 110 and plate 210. Locking nut 280 is threadedly advanced along the exterior threads 254 such that the nut engages connection portion 230 of plate 210. As shown in Fig. 2C, the enlarged shoulder on the underside of nut 280 mates with a corresponding annular ling formed in the upper portion of aperture 232. In one aspect, the extension plate 210 directly engages the previously implanted bone fixation member 120 and is spaced by the coupling member flange 252 from direct contact with previously implanted plate 110. It will be appreciated that the extension plate may be positioned at a plurality of locations with respect to plate 110 since it engages the bone bolt 120.

In use, the surgeon identifies the location of the previously implanted fixation systems 100, 150. An evaluation, generally through non-invasive imaging, is performed to determine the length of revision system needed to address the additional spinal segment(s) needing fixation. Once the initial evaluation is complete, the surgeon gains surgical access to the site within the patient and performs any decompression, fusion or other necessary procedure on the patient. If a coupling member 250 was utilized during the initial installation, then the previously implanted fixation system is ready for extension. If conventional fastener such as nut 114 was utilized on bone fastener 120, then the conventional fastener is removed. A coupling member 250 according to the present invention is then placed on the threaded post 126 and advanced along the threads by a tool (not shown) engaging drive surfaces 256 to lock plate 110 to the bone bolt 120. If necessary, a secondary tool may engage the internal drive socket of bone bolt 120 to prevent rotation of the bone screw portion within the bone of the patient. After the coupling member 250 is installed, the extension plate connection portion is positioned over the coupling member 250 and advanced toward the patient such that the coupling member extends into aperture 232 and locking projection 234 extends into slot 130 of the previously implanted plate 110. Once the extension plate is properly positioned, locking nut 280 is advanced on external threads by a tool (not shown) engaging the internal drive socket 284 of the locking nut. The extension plate 210 extends to at least vertebra V3 such that fixation portion 218 is positioned adjacent the bone. A bone fixation member 290 similar to bolt 120 is inserted through slot 220 and nut 294 is applied to lock the extension plate 210 to vertebra V3. In a similar manner, companion extension system 298 is attached to previously implanted fixation system 150.

In the illustrated embodiment, the longitudinal axis of the plate 110 is in substantial alignment with the longitudinal axis LP of plate 210. However, it is contemplated that in an alternative embodiment, the projection 234 may be positioned off the longitudinal axis alp of plate 210 such that the alignment of the extension plate 210 does not have to correspond to the alignment of the previously implanted plate.

Referring now to Figs. 3A-3C, there is shown a further embodiment of an extension system according to the present invention. Illustration of plate 110 is provided as an indication of a previously implanted fixation system 100 as shown in Fig. 1. Further description of the implanted fixation system 100 will not be described except as necessary to understand the application of the extension system to the existing system. Extension system 300 includes a plate 310, a coupling member 320 and a locking member 370. The plate 310 includes a fastening portion 318 for joining to the bone adjacent to the fixation system 100 and a coupling portion 330 for joining to the previously implanted fixation system 100. Unlike the embodiment described with respect to Figs. 1-2C, the present embodiment is intended to join directly to the plate 110 and bypass the previously implanted bone fastener. Thus, the plate 310 is provided with an enlarged bone fastener bypass portion 350 disposed between the connection portion 330 and the bone fastening portion 318. The bypass portion 350 in the slotted portion of plate 310 by opposing arcuate side walls 352 and 354 defining substantially cylindrical aperture 356.

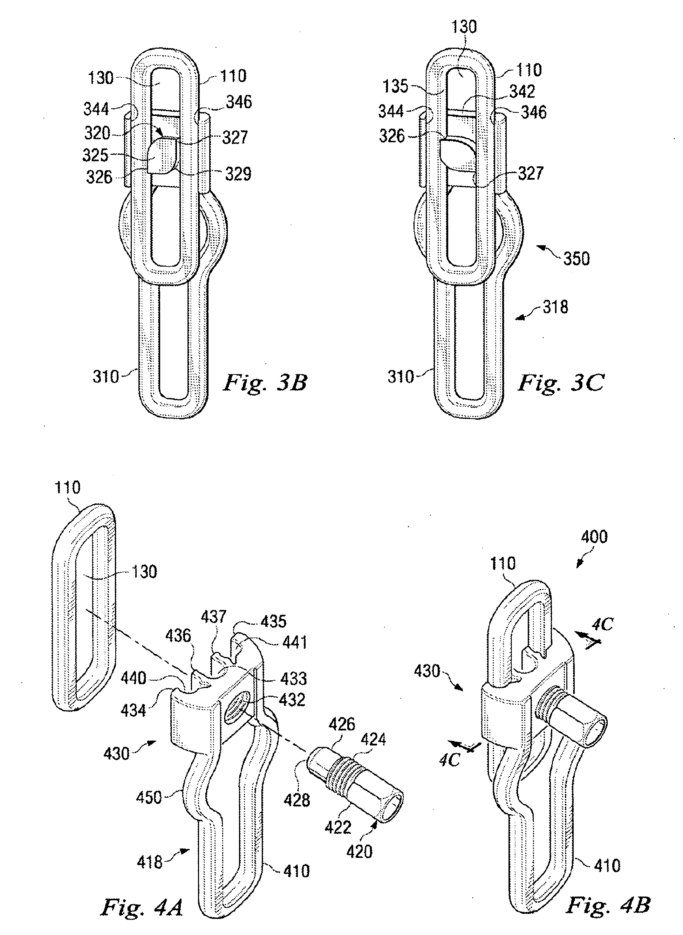

The coupling member 320 includes an enlarged head 325 and a threaded shaft 322. The enlarged head 325 includes a pair of opposing extensions 326 and 327. When the extensions are oriented in a first position as shown in Fig 3B, the head 325 is sized to pass through the slot in plate 110. When oriented in a second position as shown in Fig. 3C substantially 90 degrees rotation from the first position, the extensions 326 and 327 engage the lower surface of the plate 110 such that the coupling member head cannot pass through the slot in the plate 110. An internal socket 324 is provided in the threaded shaft such that a tool may be used to orient the head 325 and maintain the desired position as locking member 370 is tightened.

The connection portion 330 or the extension system 300 includes a substantially solid block 340 defining a central aperture 332 with a surrounding annular recess 334. The block 340 further defines a pair of flanges 344, 346 projecting from block 340 transverse to the longitudinal axis of the plate to define a channel 342 on the bottom surface. The channel 342 is configured and sized to receive the plate 110. In the illustrated embodiment, the channels is in substantial alignment with the longitudinal axis of the plate 310.

It is contemplated that the extension system 300 will be implanted as an extension of a previously implanted fixation system 100 as shown in Fig. 1. In use, the surgeon will gain surgical access to the previously implanted system. The enlarged head of the coupling member 320 will be passed through the slot in the plate 110. The plate 310 will be aligned with the plate 110 such that by pass portion is positioned over the previous implanted bone fastener device, if one exists. The threaded post 322 will be aligned with the opening 332 and the plate 310 advanced into engagement with plate 110. The flanges 344 and 346 will be positioned on either side of the previously implanted plate and the locking nut 370 will be threaded onto threaded post 322 lock the extension plate firmly to the previously implanted fixation system. At least one bone fastener is used to join the plate 310 to the bone.

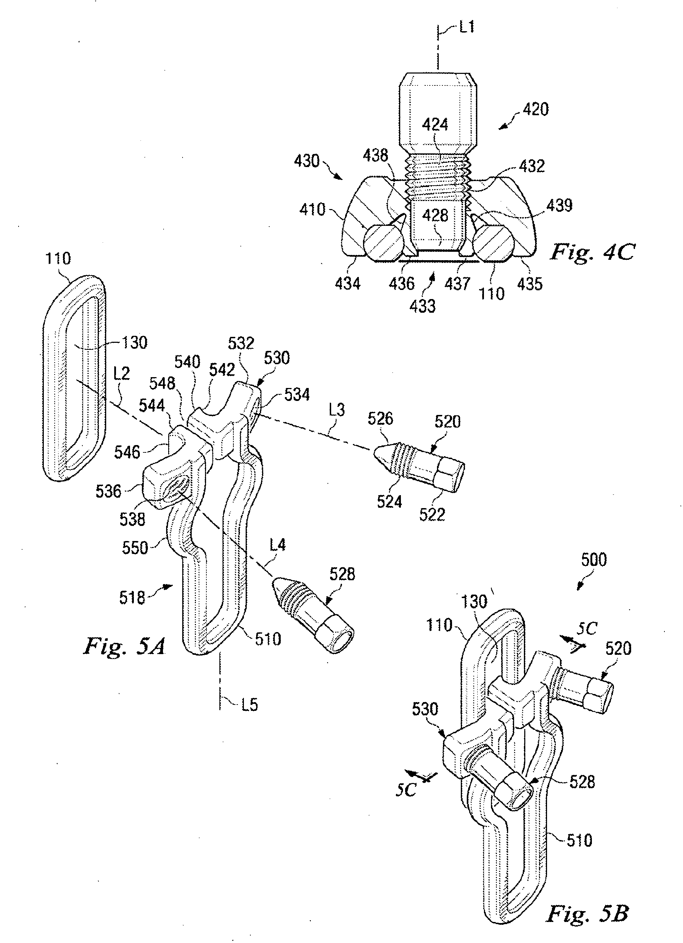

Referring now to Figs. 4A-4C, there is shown a further embodiment of an extension system according to the present invention. Extension system 400 includes a bone fastening portion 418, a fastener bypass portion 450 and a connection portion 430. Connection portion 430 includes an internally threaded aperture 432 extending transverse to the longitudinal axis of the plate and opening into a passage 433. The connection portion has a width greater than the width of plate 110 and includes a pair of external flanges 434, 435 extend substantially transverse to the longitudinal axis of the plate witch an internal distance between the ranges sufficient to receive at least a portion of plate 110. A pair of internal flange 436, 437 extend substantially parallel to external flanges 43, 435 to define plate receiving channels 440 and 441 therebetween, respectively. Internal flanges 436, 437 define passage 433 therebetween. As best seen in Fig. 4C, each of internal flanges 436, 437 have a reduced width portion adjacent their connection to plate body 410 and a sloped bearing surface adjacent passage 433 tapering to narrow the passage towards the bottom of the connection portion 430. As a result of the reduced width portion, internal flanges 436, 437 may flex inward to allow loading or the plate into channels 440, 441 and outwardly to lock the plate in position. External flanges 434, 435 each have an inner surface with a lower edge. The lower edge of the inner surface extends inwardly slightly to form a concave recess to receive a portion of the plate and cooperate in locking the plate 110 to the connection portion 430.

Locking member 420 is formed substantially as a set screw with an external driving pattern 422 and external thread form 424. Projecting cylindrical shaft 426 extend beyond thread form 424 and terminates in conical portion 428. It is contemplated that locking member 420 is a break-off set screw such that after tightening the driving portion 422 may be sheared off to lower the profile of the extension system 400.

Use of the extension system 400 is substantially the same as previously described above with respect to system 300. However, unlike system 300, extension system 400 is attached to the previously implanted fixation system 100 without any components engaging the bottom of the plate 110. Specifically, the connection portion 430 is aligned with the plate 110 with bypass portion 450 positioned adjacent any previously implanted bone fixation devices. The connection portion 430 is press fit onto the plate 110 with each side portion of the plate received in channels 440 and 441. As explained above, the infernal flanges 436 and 437 flex inward slightly to allow the plate to be seated in the channels. Locking member 420 is threadedly advanced into passage 433 along axis L1 to force conical surface 428 to against the bearing surfaces of the internal flanges. Continued advancement of the conical surface 428 against the bearing surfaces force the internal flanges toward the external flanges thereby capturing the plate within the channels. In an alternative embodiment, the internal flanges are preformed to allow the plate to be positioned within the channels and are moved there after to lock the plate in the channels.

Preferring now to Fig. 5A-5C, there is shown a further embodiment of an extension system in accordance with another aspect of the present invention. Extension system 500 includes an elongated plate 510 having a bone fastener portion 518, a connection portion 530 and a fastener bypass portion 550. Connection portion 530 includes a first lateral extension 532 extending laterally away from longitudinal axis L5. An internally threaded bore 534 is formed in lateral extension 532 extending along axis L3. In a similar manner, second lateral extension 536 has an internally threaded bore 538 extending along axis L4. The connection portion 530 is divided into independent halves by gap 548. First lateral extension also includes an internal flange 540 projecting transverse from the longitudinal axis toward the previously implanted fixation system 100. Internal projection 540 includes a external side wall having a concave recessed area terminating in tip 542 projecting laterally. Similarly, second lateral extension also includes an internal flanges 540 projecting transverse from the longitudinal axis toward the previously implanted fixation system 100, Internal projection 540 includes an external side wall having a concave recessed area terminating in tip 542 projecting laterally. Similarly, internal projection 544 includes an external side wall having a concave recessed area terminating in tip 546 projecting laterally.

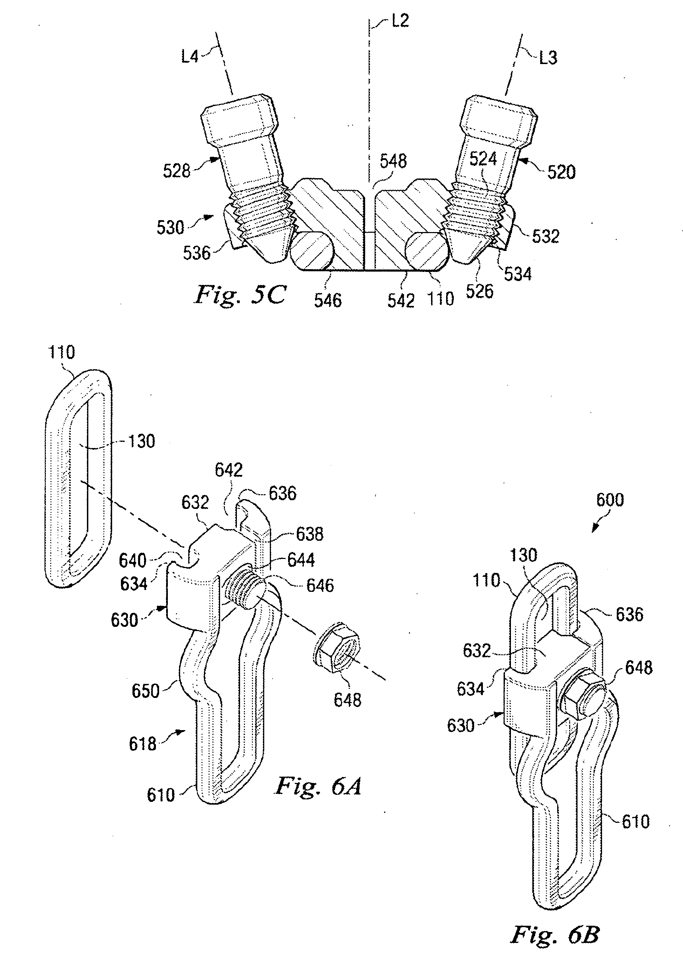

The extension system 500 is used to extend a previously implanted system 100 as described above, In operation, the user positioned in the extension system 500 in alignment with a portion of plate 110 such that bypass area 550 is aligned with a preexisting bone fixation member, if it is necessary to straddle the bone fixation member to have sufficient area to complete the connection. Either manually or with a tool, the sides of the plate 510 may be compressed to narrow the gap 548 such that the projections 542 and 546 move medially. In this compressed form, the projections may pass through the slot 130 of the plate 110 as plate 510 is advanced along axis L2 into engagement. Once the internal flanges are positioned in the slot 130, the locking members 520 and 528 may be applied. Locking member 520 has an external drive pattern, an externally threaded shaft 524 and a conical tip 526. Locking member 528 is similarly formed. As best seen in Fig. 5C, locking member 520 is advanced with threaded opening 534 along axis L3. In the illustrated embodiment, axis L3 extends at an oblique angle with respect to axis L2. Continued advancement of the set screw locking member 520 along axis L3 forces conical tip 534 against plate 110 thereby forcing the plate member medially into locking engagement with internal flange 540 and the projecting tip 542, In a similar manner, locking member 528 is advanced along axis L4, extending non parallel to axis L2, within bore 538. As the locking member advances, the tip engages the opposite side of plate 110 and forces it medially toward internal flange 544 and projection 546, thereby locking the connection assembly 530 and plate 110. If necessary, removal may be accomplished by reversing the connection members to allow movement of the plate 110 with respect to the internal flanges.

Referring now to Figs. 6A and 6b, there is shown a further embodiment of an extension assembly according to the present invention. Extension assembly 600 includes a slotted plate 610 having a bone fastener area 618, a fastener bypass area 650 and a coupling portion 630. The coupling portion 630 includes a first lateral flange 634 extending substantial transverse to the longitudinal axis of the plate 610 and a second lateral flange 636 extending substantial transverse to the longitudinal axis of the plate 610. The first and second lateral flanges 634 and 636 having medial facing surfaces spaced from each other a distance greater than the width of the plate 110 at the connection area. A central internal flange 632 projects downward toward the previously implanted fixation plate and the underlying bone. A first channels 640 is defined between lateral flange 634 and central flange 632. Similarly, a second channel 642 is defined between lateral flange 636 and central flange 632 Second flange 636 includes a projecting arm 638 that is received within a passage of coupling portion 630 and has an end that ends into channel 640. Coupling member 646 extends through aperture 644 and engages the projecting arm 638. Locking nut 648 is threaded onto threads of the coupling member to lock the projection arm 638 in relative position in comparison to the lateral flange 634.

In use, the coupling portion is positioned over plate 110 such that the plate sides extend within channels 640 and 642 as shown in Fig 6A. Either by manual force or with a compression tool, the second lateral flange 636 is urged toward the fist lateral flange 634 thereby closing charnels 640 and 642. Thus, the lateral flanges with the inwardly projecting tips at their distal end lockingly hold the plate 110 from translational and rotational movement. Locking nut 648 is threaded onto the post 646 to maintain the lateral flanges in the locking position shown in Fig. 6B.

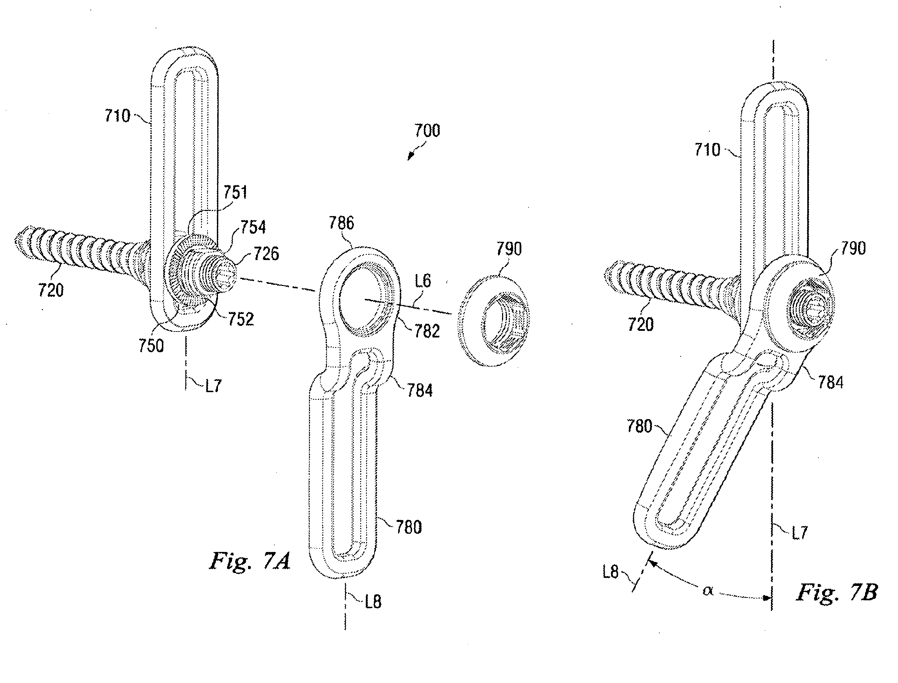

Referring now to Figs. 7A and 7B, there is shown still a further embodiment of a fixation system according to another aspect of the present invention. Plate 710 represents a previously implanted fixation system attached to the bone via bone screw 720 having a post 726 with a series of external machine threads. The plate 710 extends along longitudinal axis L7 with an upper surface extending substantially along an implant plane and the bone screw 720 extend along axis L6 extending substantially transverse to axis L7. A dual threaded coupling nut 750 has been applied to and threadedly engaged with post 726 to couple plate 710 to bone screw 720. Nut 750 includes a plurality of splines radially 752 extending along the surface of the washer flange 751 adjacent to the externally threaded post 754. In the illustrated embodiment, the washer flange 751 is integrally formed with the threaded post 754 of nut 750.

An elongated extension member 780 is provided extending, along longitudinal axis L8. In the illustrated embodiment, the extension members 780 is a slotted plate having an upper surface extending substantially along a first plane formed with a connection portion 782 extending substantially along a second plane. Extending between the slotted plate portion and the connection portion 782 is profile reduction transition area 784 sloping between the first plane and the second plane. Defined on the bottom of the connection portion 782 is a series of radially extending splines 786 substantially identical to splines 752 in size and arrangement such that they may mate with splines 752. It will be appreciated that extension member 780 may be positioned at a plurality of angular relations with respect to plate 710 such that longitudinal axis L8 may extend at an angle α with respect to longitudinal axis L7. It will be appreciated that transition area 784 is formed to permit both the bottom surface of connection portion 780 and the first plane to be in substantial alignment with the implant plane of plate 710. The extension member is locked in position by applying nut 790 to the threaded post 754. It will be appreciated that nut 790 inhibits movement of the extension plate 780 in the direction of longitudinal axis L6 while the interdigitating engagement of splines 752 with the corresponding splines 786 on the bottom of connection portion 782 inhibits rotation of plate 780 about axis L6. Thus, the embodiment of Figs. 7A and 7B provides a multi axial connection between implant 710 and extension member 780.

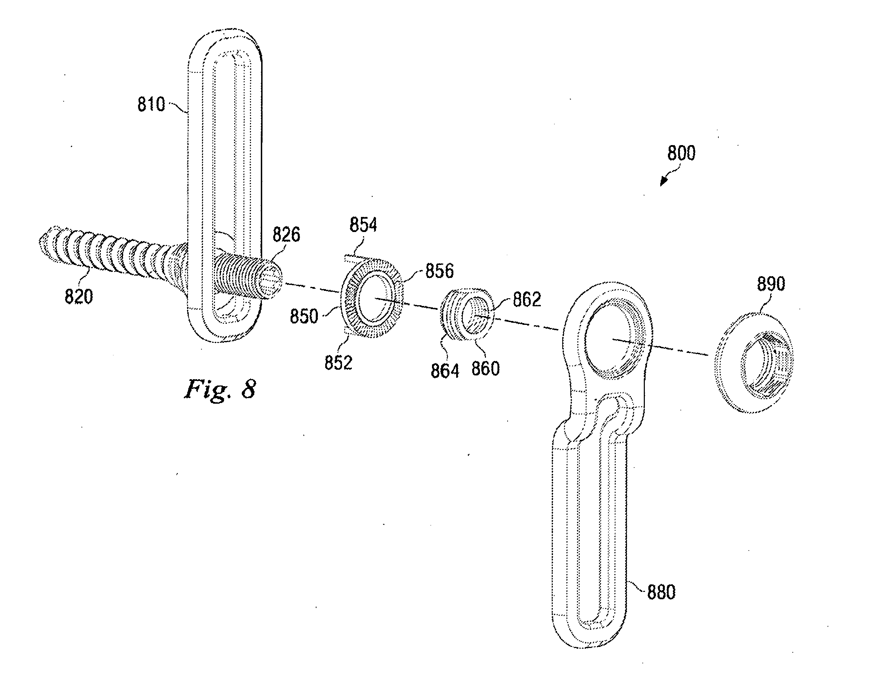

Referring now to Fig. 8, there is shown still a further embodiment of the present invention. Plate 810 represents a previously implanted fixation system attached to the bone via bone screw 820 having a post 826 with a series of external machine threads. A locking washer 850 includes a series of radially extending splines 856 projecting upwardly and a pair of downwardly projecting tabs 852 and 854. Locking washer 850 is positioned over threaded post 826 with tabs 852 and 854 extending into the slot of plate 810. A dual threaded nut 860 is advanced along threaded post 826 to engage a recessed area of washer 850 adjacent to the splines 856 to thereby lock the washer fit0 to plate 810. Internal threads 862 engage the external threads of post 826. Extension plate 880 has a series of radially extending splines corresponding to splines 856 formed on its bottom surface surrounding the mating aperture. The plate 880 is positioned over the threaded post 826 such that the splines on the bottom of the plate are matingly interdigitated with the splines 856. Locking nut 890 is applied to the threaded post 826 to lock the assembly in position. It will be appreciated that with the multi-angle coupling between the splines, plate 880 may extend at a plurality of angles with respect to plate 810. While tabs 852 and 854 are shown in the illustrated embodiment to resist rotation about the bone screw, it will be appreciate in an alternative embodiment the tabs are removed and resistance to rotation of the washer against the plate is accomplished by other forms such as compression onto the plate, a roughened surface on the washer bottom, frictional engagement or an interference fit

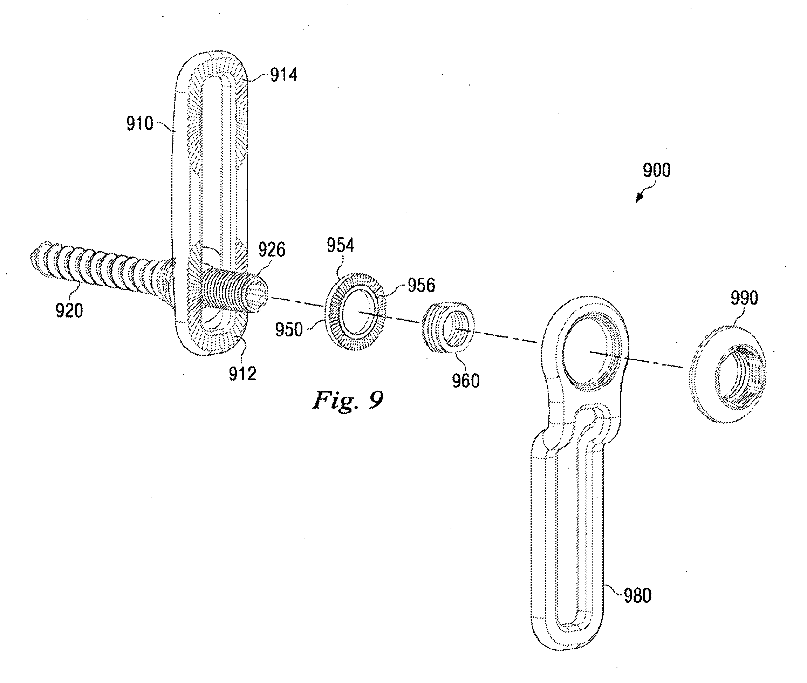

Referring now to Fig. 9, there is shown yet a further embodiment of the present invention. Plate 910 represents a previously implanted fixation system attached to the bone via bone screw 920 having a post 926 with a series of external machine threads. The upper surface of plate 910 is provided with a plurality of upwardly projecting splines 912. A locking washer 950 includes a series of radially extending splines 956 projecting upwardly and a series of downwardly projecting splines 954 configured to matingly engage splines 912. Locking washer 950 is advanced over threaded post 926 with the downwardly projecting splines extending into engagement with splines 912. A dual threaded nut 960 is advanced along threaded post 926 to engage a recessed area of washer 950 adjacent to the splines 956 to thereby lock the washer 950 to plate 910. Extension plate 980 has a series of radially extending splines corresponding to splines 956 formed on its bottom surface surrounding the mating aperture. The plate 980 is positioned over the threaded post 926 such that the splines on the bottom of the plate are matingly interdigitated with the splines 956. Locking nut 990 is applied to the threaded post 926 to lock the assembly in position. In an alternative embodiment, radially projecting splines are replaced with a series of ridges extending transverse to the longitudinal axis of the plate or with a knurled surface. The bottom of the washer, or the extension plate itself, is formed with a mating series of ridges or knurling.

Although only a few exemplary embodiments have been described in detail above, those skilled in the art will readily appreciate that many modifications are possible in the exemplary embodiments without materially departing from the novel teachings and advantages of this disclosure.

The extension systems described above are formed of any suitable biocompatible material. It is contemplated that the extension system is formed of substantially the same material as the previously implanted fixation system. Examples of suitable materials include, but are provided without limitation to the use of alternative materials to form extension systems, metals such stainless steel and titanium, composites, ceramics, plastics, and polymers. Further, while the illustrated embodiments have shown a number of components integrally formed with the elongated fixation member or plate, it is contemplated that such components may separately formed and joined by any suitable connection.

Although the previously implanted system has been described for the purposes of illustration as a plate and pedicle screw system, it is contemplated that the present invention may be used with rod and screw systems, other plate and screw systems, and any spinal fixation or stabilization system to which the extension systems of the present disclosure may be connected.

It is understood that all spatial references, such as "top," "inner," "outer," "bottom," "left," "right," "anterior," "posterior," "superior," "inferior," "medial," "lateral," "upper," and "lower" are for illustrative purposes only and can be varied within the scope of the disclosure.

## Claims

1. A system for extending a first, implanted spinal fixation element (110) to one or more additional adjacent vertebrae while leaving the first, implanted spinal fixation element in place, the system comprising:
an elongated extension member (210) having a longitudinal axis extending between a coupling end portion (230) and a bone engagement end portion (218), said elongated extension member having a length between said coupling end portion and said bone engagement portion capable of extending from said first, implanted spinal fixation element to at least one additional vertebra;
said bone engagement end portion having an elongate slot (220) therein; and a coupling element (250) connectable to said coupling end portion and movable from an unlocked position allowing movement between said elongated extension member (210) and said first implanted spinal fixation element (110), and a locked position preventing movement between said elongated extension member and said first implanted spinal fixation element;
**characterised in that** said coupling end of the elongated extension member includes means (234) for inhibiting rotation of the elongated extension member about the coupling element (250).

2. The system of claim 1, wherein said means for inhibiting rotation is a pair of flanges (344, 346; 436, 437; 540, 544; 634, 636) extending substantially transverse to said longitudinal axis.

3. The system of claim 2, wherein said first, implanted spinal fixation element (110) includes an internal opening (130) and said pair of flanges (436,437) are configured for placement at least in part within said opening.

4. The system of claim 3, wherein said pair of flanges (436,437) are movable from a first insertion configuration having a first width to a second locking configuration having a second width, said second width greater than said first width, said coupling element operable to move said pair of flanges from said first insertion configuration to said second locking configuration.

5. The system of claim 4, wherein said coupling element is a set screw (420) positioned substantially between said pair of flanges (436,437), preferably, wherein said first, implanted spinal fixation element (110) has an exterior width and wherein said elongated extension member (410) further includes a pair of external flanges (434,435) spaced by a first distance, said first distance equal to or greater than said exterior width such that said pair of external flanges may be positioned about at least a portion of said first, implanted spinal fixation element (110) preferably, wherein said pair of external flanges (434,435) are positioned adjacent said pair of flanges (436,437) extendable into the internal opening, preferably, wherein said pair of external flanges are substantially co-located with said pair of flanges along the longitudinal axis.

6. The system of claim 3, wherein said first implanted spinal fixation element (110) is a plate having an exterior surface and said coupling element (520) is a compression member positioned to contact said exterior surface and urge said internal opening into contact with at least a portion of said pair of flanges (540,544), preferably, wherein said compression member is positioned on a first side of said plate and further including a second compression member (528) positioned on a second side of said plate opposite said first side, said first and second compression members cooperating to engage said pair of flanges with said internal opening (130) in the locked position.

7. The system of claim 2, wherein said pair of flanges (344,346) extend around a portion of an exterior surface of the implanted spinal fixation element (110), preferably, further including a set screw to lock the fixation element to at least one flange, preferably, wherein the pair of flanges include at least one movable portion, and further including a retention member (370) to hold the movable portion in a locked position.

8. The system of claim 5, wherein said pair of external flanges (434,435) each include a recessed surface for engaging the first implanted spinal fixation element (110).

9. The system of claim 1, wherein said means for inhibiting rotation includes at least one spline (786) formed on said coupling end (782), preferably, wherein said coupling element (750) includes at least one locking spline (752) for mating with said at least one spline on the coupling end.

10. The system of claim 1, wherein said means for inhibiting rotation includes at least one locking projection (234) extending substantially transverse to said longitudinal axis for engaging the first implanted spinal fixation element (110);
wherein said coupling element (250) is adjacent said at least one locking projection (234), said coupling element being moveable from said unlocked position allowing the elongated extension member (210) to be applied to the first, implanted spinal fixation element (110) to said locked position locking the elongated extension member to the first, implanted spinal fixation element;
the system further comprising a bone engagement fastener (120) capable of joining said bone engagement end portion to at least one additional adjacent vertebra.

11. The system of claim 10, wherein said elongated extension member (210) includes a bottom bone facing surface and said at least one locking projection (234) extends from a portion of said bone facing surface, preferably, wherein said elongated extension member has a top surface opposite said bottom surface with a thickness therebetween, wherein said at least one locking projection (234) has a length greater than said thickness.

12. The system of claim 10, wherein said first, implanted spinal fixation element (110) is a plate with a slot (130) and said at least one locking projection (234) is configured to extend at least in part within the slot.

13. The system of claim 12, wherein said at least one locking projection is a single flange substantially aligned with said longitudinal axis having a flange width substantially matching a slot width of the plate; or wherein said at least one locking projection includes an interior flange extending within the slot and said coupler is a set screw (420) forcing a portion of the plate against a lateral face of the interior flange (436,437).

14. The system of claim 12, wherein the plate (110) has an exterior with an exterior width and said at least one locking projection includes a pair of exterior flanges (344,346) projecting from said coupling end (330) and spaced apart by an interior distance equal to or greater than said exterior width such that the pair of flanges may be mounted to the plate about a portion of the plate exterior.

15. The system of claim 14, wherein said coupler (320) extends through said slot and through said coupling end portion (330) to join said plate (110) and said elongated extension member; or further including at least one interior flange facing said pair of exterior flanges (344,346) to form at least one channel to receive the slotted plate.

## Patentansprüche

1. System zum Erweitern eines ersten implantierten Wirbelsäulenfixationselements (110) zu einem oder mehreren benachbarten Wirbeln, während das erste implantierte Wirbelsäulenfixationselement an der Verwendungsposition bleibt, wobei das System folgendes umfasst:
ein elongiertes Verlängerungselement (210) mit einer Längsachse, die sich zwischen einem Kopplungsendstück (230) und einem Knocheneingriffsendstück (218) erstreckt, wobei das genannte elongierte Verlängerungselement eine Länge zwischendem genannten Kopplungsendstück und dem genannten Knocheneingriffsendstück aufweist, die eine Verlängerung von dem genannten ersten implantierten Wirbelsäulenfixationselement zu mindestens einem weiteren Wirbel ermöglicht;
wobei das genannte Knocheneingriffsendstück darin einen Langschlitz(220) aufweist; und
ein Kopplungselement (250), das mit dem genannten Kopplungsendstück verbunden und von einer entriegelten Position beweglich ist, so dass eine Bewegung zwischen dem genannten elongierten Verlängerungselement (210) und dem genannten ersten implantierten Wirbelsäulenfixationselement ermöglicht ist, und einer verriegelten Position, die eine Bewegung zwischendem genannten elongierten Verlängerungselement und dem genannten ersten implantierten Wirbelsäulenfixationselement verhindert;
**dadurch gekennzeichnet, dass** das genannte Kopplungsende des elongierten Verlängerungselements eine Einrichtung (234) zum Verhindern einer Rotation des elongierten Verlängerungselements um das Kopplungselement aufweist.

2. System nach Anspruch 1, wobei es sich bei der genannten Einrichtungzum Verhindern einer Rotation um ein Flanschpaar (344, 346; 436, 437; 540, 544; 634, 636) handelt, das sich im Wesentlichen transversal zu der genannten Längsachse erstreckt.

3. System nach Anspruch 2, wobei das genannte erste implantierte Wirbelsäulenfixationselement (110) eine innere Öffnung (130) aufweist, und wobei das genannte Flanschpaar (436, 437) zur Platzierung zumindest teilweise innerhalb des genannten Gehäuses konfiguriert ist.

4. System nach Anspruch 3, wobei das genannte Flanschpaar (436, 437) von einer ersten Einführungskonfiguration mit einer ersten Breite an eine zweite Verriegelungsposition mit einer zweiten Breite beweglich ist, wobei die genannte zweite Breite größer ist als die genannte erste Breite, wobei das genannte Kopplungselement so bedient werden kann, dass es das genannte Flanschpaar von der genannten ersten Einführungskonfiguration an die genannte zweite Verriegelungskonfiguration bewegt.

5. System nach Anspruch 4, wobei es sich bei dem genannten Kopplungselement um eine Stellschraube (420) handelt, die vorzugsweise im Wesentlichen zwischendem genannten Flanschpaar (436, 437) positioniert ist, wobei das genannt erste implantierte Wirbelsäulenfixationselement (110) eine äußereBreite aufweist, und wobei das genannte elongierte Verlängerungselement (410) ferner ein Paar äußerer Flansche (434, 435) aufweist, die mit einem ersten Zwischenabstand zueinander räumlich getrennt angeordnet sind, wobei der genannte erste Zwischenabstand größer oder gleich der genannten äußeren Breite ist, so dass das genannte Paar äußerer Flansche vorzugsweise zumindest um ein Teilstückdes genannten ersten implantierten Wirbelsäulenfixationselements (110) positioniert werden kann, wobei das genannte Paar äußerer Flansche (434, 435) angrenzend an das genannte Flanschpaar (436, 437) positioniert ist, vorzugsweise erweiterbar in die innere Öffnung, wobei das genannte Paar äußerer Flansche im Wesentlichen eine Kolokation mit dem genannten Flanschpaar entlang der Längsachse aufweist.

6. System nach Anspruch 3, wobei es sich bei dem genannten ersten implantierten Wirbelsäulenfixationselement (110) um eine Platte mit einer äußeren Oberfläche handelt, und wobei es sich bei dem genannten Kopplungselement (520) um ein Kompressionselement handelt, das so positioniert ist, dass es die genannte äußereOberfläche berührt und die genannte innere Öffnung vorzugsweise in Kontakt mit zumindest einem Teilstückdes genannten Flanschpaares (540, 544) drängt, wobei das genannte Kompressionselement auf einer ersten Seite der genannten Platte positioniert ist, und ferner mit einem zweiten Kompressionselement (528), das auf einer zweiten Seite der genannten Platte gegenüber der genannten ersten Seite positioniert ist, wobei die genannten ersten und zweiten Kompressionselemente so zusammenwirken, dass sie einen Eingriff des genannten Flanschpaares mit der genannten inneren Öffnung (130) an der verriegelten Position vorsehen.

7. System nach Anspruch 2, wobei sich das genannte Flanschpaar (344, 346) vorzugsweise um ein Teilstück einer äußeren Oberfläche des implantierten Wirbelsäulenfixationselements (110) erstreckt, ferner mit einer Stellschraube zum Verriegeln des Fixationselements vorzugsweise an mindestens einem Flansch, wobei das Flanschpaar mindestens ein bewegliches Teilstückaufweist, und ferner mit einem Sicherungselement (370) zum Halten des beweglichen Teilstücksan einer verriegelten Position.

8. System nach Anspruch 5, wobei in dem genannten Paar äußerer Flansche (434, 435) jeder Flansch eine ausgesparte Oberfläche für einen Eingriff mit dem ersten implantierten Wirbelsäulenfixationselement (110) aufweist.

9. System nach Anspruch 1, wobei die genannte Einrichtung zum Verhindern einer Rotation mindestens einen Keil (7869 aufweist, der vorzugsweise an dem genannten Kopplungsende (782) ausgebildet ist, wobei das genannte Kopplungselement (750) mindestens einen Verriegelungskeil (752) aufweist, der mit dem genannten mindestens einen Keil an dem Kopplungsende zusammenpasst.

10. System nach Anspruch 1, wobei die genannte Einrichtung zum Verhindern einer Rotation mindestens einen Verriegelungsvorsprung (234) aufweist, der sich im Wesentlichen transversal zu der genannten Längsachse erstreckt, um mit dem ersten implantierten Wirbelsäulenfixationselement (110) einzugreifen;
wobei das genannte Kopplungselement (250) angrenzend an den genannten mindestens einen Verriegelungsvorsprung (234) angeordnet ist, wobei das genannten Kopplungselement aus der genannten entriegelten Position beweglich ist, so dass das elongierte Verlängerungselement (210) auf das erste implantierte Wirbelsäulenfixationselement (110) an die genannte entriegelte Position angewandt werden kann, wobei das elongierte Verlängerungselement an dem ersten implantierten Wirbelsäulenfixationselement verriegelt wird;
wobei das System ferner eine Knocheneingriffsbefestigungseinrichtung (120) umfasst, welche das genannte Knocheneingriffsendstück mit mindestens einem zusätzlichen benachbarten Wirbel verbinden kann.

11. System nach Anspruch 10, wobei das genannte elongierte Verlängerungselement (210) eine untere Oberfläche mit Knochenausrichtung aufweist, und wobei sich der genannte mindestens eine Verriegelungsvorsprung (234) vorzugsweise von einem Teilstückder genannten Oberfläche mit Knochenausrichtung erstreckt, wobei das genannte elongierte Verlängerungselement eine obere Oberfläche entgegengesetzt zu der genannten unteren Oberfläche mit einer dazwischen liegenden Dicke aufweist, wobei der genannte mindestens eine Verriegelungsvorsprung (234) eine Länge aufweist, die größer ist als dessen Dicke.

12. System nach Anspruch 10, wobei es sich bei dem genannten ersten implantierten Wirbelsäulenfixationselement (110) um eine Platte mit einem Schlitz (130) handelt, und wobei der genannte mindestens eine Verriegelungsvorsprung (234) so konfiguriert ist, dass er sich zumindest teilweise in dem Schlitz erstreckt.

13. System nach Anspruch 12, wobei es sich bei dem genannten mindestens einen Verriegelungsvorsprung um einen einzelnen Flansch handelt, der im Wesentlichen mit der genannten Längsachse ausgerichtet ist, mit einer Flanschbreite, die im Wesentlichen einer Schlitzbreite der Platte entspricht; oder wobei der genannte mindestens eine Verriegelungsvorsprung einen inneren Flansch aufweist, der sich in dem Schlitz erstreckt, und wobei es sich bei der genannten Kopplungseinrichtung um eine Stellschraube (420) handelt, welche ein Teilstückder Platte gegen eine laterale Seite des inneren Flansches (436, 437) drückt.

14. System nach Anspruch 12, wobei die Platte (110) eine Außenseite mit einer äußeren Breite aufweist, und wobei der genannte mindestens eine Verriegelungsvorsprung ein Paar äußerer Flansche (344, 346) aufweist, die von dem genannten Kopplungsende (330) vorstehen und zwischeneinander einen inneren Zwischenabstand aufweisen, der größer oder gleich der genannten äußeren Breite ist, so dass das Flanschpaar an der Platte um ein Teilstückder Plattenaußenseite angebracht werden kann.

15. System nach Anspruch 14, wobei sich die genannte Kopplungseinrichtung (320) durch den genannten Schlitz und durch das genannte Kopplungsendstück (330) erstreckt, um die genannte Platte (110) und das genannte elongierte Verlängerungselement zu verbinden; oder ferner mit mindestens einem inneren Flansch, der zu dem genannten Paar äußerer Flansche (344, 346) ausgerichtet ist, so dass zumindest ein Kanal für die Aufnahme der geschlitzten Platte gebildet wird.

## Revendications

1. Système pour étendre un premier élément de fixation vertébrale implanté (110) vers une ou plusieurs vertèbres adjacentes supplémentaires tout en laissant le premier élément de fixation vertébrale implanté en place, le système comprenant :
un élément d'extension allongé (210) ayant un axe longitudinal s'étendant entre une partie d'extrémité de couplage (230) et une partie d'extrémité de mise en prise d'os (218), ledit élément d'extension allongé ayant une longueur comprise entre ladite partie d'extrémité de couplage et ladite partie de mise en prise d'os capable de s'étendre dudit premier élément de fixation vertébrale implanté vers moins une vertèbre supplémentaire ;
ladite partie d'extrémité de mise en prise d'os ayant une fente allongée (220) à l'intérieur ; et
un élément de couplage (250) pouvant être connecté à ladite partie d'extrémité de couplage et étant mobile d'une position déverrouillée permettant un mouvement entre ledit élément d'extension allongé (210) et ledit premier élément de fixation vertébrale implanté (110), et une position verrouillée empêchant tout mouvement entre ledit élément d'extension allongé et ledit premier élément de fixation vertébrale implanté ;
**caractérisé en ce que** ladite extrémité de couplage de l'élément d'extension allongé comprend des moyens (234) pour empêcher la rotation de l'élément d'extension allongé autour de l'élément de couplage (250).

2. Système selon la revendication1, dans lequel lesdits moyens pour empêcher la rotation sont une paire de brides (344, 346 ; 436, 437 ; 540, 544 ; 634, 636) s'étendant sensiblement transversalement audit axe longitudinal.

3. Système selon la revendication2, dans lequel ledit premier élément de fixation vertébrale implanté (110) comprend une ouverture interne (130) et ladite paire de brides (436, 437) est configurée pour un placement au moins en partie dans ladite ouverture.

4. Système selon la revendication3, dans lequel ladite paire de brides (436, 437) est mobile d'une première configuration d'insertion ayant une première largeur à une seconde configuration de verrouillage ayant une seconde largeur, ladite seconde largeur étant supérieure à ladite première largeur, ledit élément de couplage pouvant fonctionner pour déplacer ladite paire de brides de ladite première configuration d'insertion à ladite seconde configuration de verrouillage.

5. Système selon la revendication4, dans lequel ledit élément de couplage est une vis de pression (420) positionnée sensiblement entre ladite paire de brides (436, 437), de préférence, dans lequel ledit premier élément de fixation vertébrale implanté (110) a une largeur extérieure et dans lequel ledit élément d'extension allongé (410) comprend en outre une paire de brides externes (434, 435) espacées d'une première distance, ladite première distance étant égale ou supérieureà ladite largeur extérieure de sorte que ladite paire de brides externes peut être positionnée autour d'au moins une partie dudit premier élément de fixation vertébrale implanté (110) de préférence, dans lequel ladite paire de brides externes (434, 435) est positionnée de façon adjacente à ladite paire de brides (436, 437) pouvant s'étendre dans l'ouverture interne, de préférence, dans lequel ladite paire de brides externes est sensiblement colocalisée avec ladite paire de brides le long de l'axe longitudinal.

6. Système selon la revendication3, dans lequel ledit premier élément de fixation vertébrale implanté (110) est une plaque ayant une surface extérieure et ledit élément de couplage (520) est un élément de compression positionné pour entrer en contact avec ladite surface extérieure et pousser ladite ouverture interne en contact avec au moins une partie de ladite paire de brides (540, 544), de préférence, dans lequel ledit élément de compression est positionné sur un premier côté de ladite plaque et comprenant en outre un second élément de compression (528) positionné sur un second côté de ladite plaque opposée audit premier côté, lesdits premier et second éléments de compression coopérant pour mettre en prise ladite paire de brides avec ladite ouverture interne (130) en position verrouillée.

7. Système selon la revendication2, dans lequel ladite paire de brides (344, 346) s'étend autour d'une partie d'une surface extérieure de l'élément de fixation vertébrale implanté (110), de préférence, comprenant en outre une vis de pression pour verrouiller l'élément de fixation sur au moins une bride, de préférence, dans lequel la paire de brides inclut au moins une partie mobile, et comprenant en outre un élément de rétention (370) pour tenir la partie mobile en position verrouillée.

8. Système selon la revendication5, dans lequel ladite paire de brides externes (434, 435) comprend chacune une surface en retrait pour venir en prise avec le premier élément de fixation vertébrale implanté (110).

9. Système selon la revendication1, dans lequel lesdits moyens pour empêcher la rotation comprennent au moins une cannelure (786) formée sur ladite extrémité de couplage (782), de préférence, dans lequel ledit élément de couplage (750) comprend au moins une cannelure de verrouillage (752) pour s'accoupler avec ladite au moins une cannelure sur l'extrémité de couplage.

10. Système selon la revendication1, dans lequel lesdits moyens pour empêcher la rotation comprennent au moins une saillie de verrouillage (234) s'étendant sensiblement transversalement audit axe longitudinal pour venir en prise avec le premier élément de fixation vertébrale implanté (110) ;
dans lequel ledit élément de couplage (250) est adjacent à ladite au moins une saillie de verrouillage (234), ledit élément de couplage étant mobile de ladite position déverrouillée permettant à l'élément d'extension allongé (210) d'être appliqué au premier élément de fixation vertébrale implanté (110) à ladite position verrouillée bloquant l'élément d'extension allongé sur le premier élément de fixation vertébrale implanté ;
le système comprenant en outre un dispositif de fixation de mise en prise d'os (120) capable de joindre ladite partie d'extrémité de mise en prise d'os à au moins une vertèbre adjacente supplémentaire.

11. Système selon la revendication 10, dans lequel ledit élément d'extension allongé (210) comprend une surface faisant face à l'os inférieure et ladite au moins une saillie de verrouillage (234) s'étend depuis une partie de ladite surface faisant face à l'os, de préférence, dans lequel ledit élément d'extension allongé a une surface supérieureopposée à ladite surface inférieure avec une épaisseur entre elles, dans lequel ladite au moins une saillie de verrouillage (234) a une longueur supérieureà ladite épaisseur.

12. Système selon la revendication10, dans lequel ledit premier élément de fixation vertébrale implanté (110) est une plaque avec une fente (130) et ladite au moins une saillie de verrouillage (234) est configurée pour s'étendre au moins en partie à l'intérieur de la fente.

13. Système selon la revendication 12, dans lequel ladite au moins une saillie de verrouillage est une bride unique sensiblement alignée avec ledit axe longitudinal ayant une largeur de bride correspondant sensiblement à une largeur de fente de la plaque ; ou dans lequel ladite au moins une saillie de verrouillage comprend une bride intérieure s'étendant à l'intérieur de la fente et ledit coupleur est une vis de pression (420) forçant une partie de la plaque contre une face latérale de la bride intérieure (436, 437).

14. Système selon la revendication 12, dans lequel la plaque (110) a un extérieur avec une largeur extérieure et ladite au moins une saillie de verrouillage comprend une paire de brides extérieures (344, 346) faisant saillie de ladite extrémité de couplage (330) et espacées par une distance intérieure égale ou supérieureà ladite largeur extérieure de sorte que la paire de brides peut être montée sur la plaque autour d'une partie de l'extérieur de la plaque.

15. Système selon la revendication14, dans lequel ledit coupleur (320) s'étend à travers ladite fente et à travers ladite partie d'extrémité de couplage (330) pour joindre ladite plaque (110) et ledit élément d'extension allongé ; ou comprenant en outre au moins une bride intérieure faisant face à ladite paire de brides extérieures (344, 346) pour former au moins un canal pour recevoir la plaque fendue.
